# Europäisches Patentamt
## European Patent Office
### Office européen des brevets

(19)

(11) Veröffentlichungsnummer: **0 135 023**
**B1**

(12) **EUROPÄISCHE PATENTSCHRIFT**

(45) Veröffentlichungstag der Patentschrift:
21.09.88

(21) Anmeldenummer: **84108367.8**

(22) Anmeldetag: **17.07.84**

(51) Int. Cl.⁴: **C 12 Q 1/04,** C 12 Q 1/18

(54) Verfahren zur Empfindlichkeitsprüfung von Bakterien.

(30) Priorität: **02.08.83 DE 3327839**

(43) Veröffentlichungstag der Anmeldung:
**27.03.85 Patentblatt 85/13**

(45) Bekanntmachung des Hinweises auf die Patenterteilung:
**21.09.88 Patentblatt 88/38**

(84) Benannte Vertragsstaaten:
**BE CH DE FR GB IT LI NL SE**

(56) Entgegenhaltungen:
**EP-A-0 018 825
EP-A-0 054 001
EP-A-0 091 837
DE-A-2 930 394**

**CHEMICAL ABSTRACTS, Band 78, Nr. 11, 19. März 1973, Seite 85, Nr. 67540e, Columbus, Ohio, US; L.G. BURMAN u.a.: "Murein and the outer penetration barrier of Escherichia coli K12, Proteus mirabilis, and Pseudomonas aeruginosa"**

(73) Patentinhaber: **Merck Patent Gesellschaft mit beschränkter Haftung, Frankfurter Strasse 250, D-6100 Darmstadt (DE)**

(72) Erfinder: **Kappner, Manfred, Dr., Lärchenweg 11, D-6110 Dieburg (DE)**
Erfinder: **Metz, Harald, Dr., Am Landbach 8, D-6101 Bickenbach (DE)**

EP 0 135 023 B1

## Beschreibung

Die Erfindung betrifft ein Verfahren zur Empfindlichkeitsprüfung von Bakterien gegen Antibiotika mit Primärwirkung in der Mureinbiosynthese.

Vor einer Antibiotikumtherapie sollte nach Möglichkeit die Empfindlichkeit des Krankheitserregers gegen verschiedene Antibiotika bestimmt werden. Die heute verwendeten Testmethoden zur Empfindlichkeitsprüfung beruhen meist auf einem Nachweis der Wachstumshemmung von Mikroorganismenreinkulturen in Gegenwart des Antibiotikums.

Die antibiotische Wirkung ist in einem in-vitro-Empfindlichkeitstest von vielen Faktoren abhängig, z. B. von der Wachstumsphase der Mikroorganismen, dem Nährmedium, der Populationsdichte. Standardmethoden für eine Empfindlichkeitsprüfung von Mikroorganismen gegen Antibiotika sind der Verdünnungstest in Agar bzw. einer Bouillon und der Agardiffusionstest.

Die Auswertung der Tests erfolgt in der Praxis meist durch visuelle Untersuchung der Testansätze auf Mikroorganismenwachstum, z. B. nach 24 Stunden Bebrütung durch Bestimmung der Trübung im Verdünnungsreihentest oder des Hemmhofdurchmessers im Agardiffusionstest. Die Analysenzeiten im Verdünnungsreihentest können durch moderne sensitive Analysenverfahren wie Impedanzmessung oder Laser-Nephelometrie auf meist 4 - 8 Stunden verkürzt werden. Die entsprechenden Analysengeräte sind jedoch in der Regel kostspielig, so daß ein Einsatz nur in Laboratorien mit einem großen Probenanfall gerechtfertigt ist; bei durchschnittlichen Analysenzeiten von 4 - 8 Stunden liegt das Ergebnis häufig erst nach Dienstschluß vor, so daß der Vorteil der verkürzten Analysenzeit durch verzögerte Informationsweiterleitung wieder zunichte gemacht wird.

Aus der EP-A-54001 ist eine Methode zur Prüfung der Empfindlichkeit von Bakterien gegen Antibiotika bekannt, die auf einer positiven Korrelation der Fluoreszenzaktivität und der Anzahl der Mikroorganismen beruht, d. h. die Fluoreszenz dient als Wachstumsindikator. Enzymaktivitäten und damit auch Fluoreszenzaktivitäten, die bei intakten Mikroorganismen als Wachstumsindikatoren dienen, sind jedoch für den Nachweis der Empfindlichkeit von Bakterien gegen Antibiotika mit Primärwirkort in der Mureinbiosynthese nicht geeignet; dazu können nur cytoplasmatische Enzymaktivitäten verwendet werden, die bei intakter Zellwand nicht nachzuweisen sind.

Für die Praxis besteht deshalb ein dringendes Bedürfnis nach einem Empfindlichkeitstest, der sehr schnell Ergebnisse liefert und der ohne allzu kostspieligen apparativen Aufwand auch in kleineren Laboratorien durchgeführt werden kann. Schnelle Empfindlichkeitstest von Mikroogranismen gegen Chemotherapeutika ermöglichen einen gezielteren Einsatz der Antibiotika, was z. B. zur Verkürzung der Behandlungszeit des Patienten und zur Eindämmung der Selektionierung multiresistenter Bakterienstämme führt.

Zu den Antibiotika, deren primärer Wirkort die Mureinbiosynthese ist, zählen neben den therapeutisch sehr interessanten Penicillinen und Cephalosporinen beispielsweise Cycloserin, Vancomycin und Bacitracin. Penicilline und Cephalosporine sind chemisch durch das Vorliegen eines β-Lactamringes gekennzeichnet. Das Resistenzverhalten von gramnegativen Bakterien gegen β-Lactamantibiotika ist unterschiedlich von dem grampositiver Organismen. Die Mehrzahl der grampositiven Bakterien produziert relativ große Mengen an extracellulären ß-Lactamasen, die den β-Lactamring aufspalten und somit die β-Lactamantibiotika inaktivieren können. Für die Empfindlichkeit von gramnegativen Bakterien gegen β-Lactamantibiotika ist häufig eine Eindringungsresistenz von wesentlich größerer Bedeutung als eine durch β-Lactamasen bedingte Resistenz. Die zellgebundenen β-Lactamasen von gramnegativen Bakterien werden in wesentlich geringeren Konzentrationen gebildet als die extracellulären β-Lactamasen bei grampositiven Bakterien. Bei gramnegativen Bakterien besteht oft keine Korrelation zwischen der minimalen Hemmkonzentration der β-Lactamantibiotika und der β-Lactamaseaktivität. β-Lactamase-positive Bakterien sind sowohl bei den β-Lactamantibiotika-empfindlichen als auch -unempfindlichen gramnegativen Bakterien zu finden. Die bekannten β-Lactamaseschnelltests besitzen somit für sich allein keine Bedeutung zur Empfindlichkeitsprüfung von z. B. Enterobacteriaceae gegen β-Lactamantibiotika.

Der Erfindung liegt die Aufgabe zugrunde, ein einfaches Verfahren und Schnelltestsystem zur Empfindlichkeitsprüfung von Bakterien gegen Antibiotika mit Primärwirkort in der Mureinbiosynthese mit Analysenzeiten deutlich unter 4 Stunden zu entwickeln.

Gegenstand der Erfindung ist ein Verfahren zur Empfindlichkeitsprüfung von Bakterien gegen Antibiotika mit Primärwirkung in der Mureinbiosynthese, das dadurch gekennzeichnet ist, daß die in Gegenwart von Antibiotikum und Enzymsubstrat aus den Bakterien freigesetzten cytoplasmatischen Enzymaktivitäten direkt bestimmt werden.

Ein Mittel zur Empfindlichkeitsprüfung von Bakterien gegen Antibiotika mit Primärwirkung in der Mureinbiosynthese, enthält im wesentlichen ein gepuffertes Nährmedium mit versschiedenen Antibiotikumkonzentrationen, mindestens ein Enzymsubstrat und gegebenenfalls mindestens ein Coenzym sowie gegebenenfalls mindestens einen Redoxindikator.

Das erfindungsgemäße Verfahren ermöglicht durch einfache und schnelle Aktivitätsmessung cytoplasmatischer Enzyme, den Einfluß von

2

Antibiotika auf die Mureinbiosynthese direkt innerhalb eines einzigen Generationszyklus des Bakteriums zu erfassen, z. B. eine Empfindlichkeitsprüfung von Bakterien gegen β-Lactamantibiotika innerhalb von 30 - 60 Minuten durchzuführen. Eine Empfindlichkeit des Bakteriums gegen das Antibiotikum kann mit dem erfindungsgemäßen Verfahren schneller als mit bekannten Verfahren nachgewiesen werden, bei denen meist die Wachstumsrate der Bakterien in Gegenwart des Antibiotikums verfolgt wird; bei diesen Verfahren sind jedoch Inkubationszeiten notwendig, die mehreren Generationszyklen des Bakteriums entsprechen.

Als Inoculum ist nach dem erfindungsgemäßen Verfahren eine einzige etwa 1 mm große Bakterienkolonie von einem festen Nährboden ausreichend, um den Empfindlichkeitstest durchzuführen. Das Prinzip des erfindungsgemäßen Empfindlichkeitstests von Bakterien gegen Antibiotika ist der direkte Nachweis einer Schädigung wachsender Bakterien in Gegenwart eines Antibiotikums mit Primärwirkort in der Mureinbiosynthese. Bei gestörter Mureinbiosynthese liegt ein unvollständiges, d. h. bruchstückhaft ausgebildetes Mureingerüst vor. Kann das Mureingerüst dem osmotischen Druck der Bakterien in einem hypotonischen Medium nicht den entsprechenden Gegendruck bieten, platzen die Zellen und intracelluläre Enzyme werden freigesetzt. Die in Gegenwart verschiedener Antibiotikumkonzentrationen freigesetzten cytoplasmatischen Enzymaktivitäten sind ein zuverlässiges Maß für die Empfindlichkeit der Bakterien gegen das Antibiotikum. Das Platzen der Zellen bei nur leicht geschädigtem Mureingerüst kann durch eine geringe Detergenzkonzentration gefördert werden. Unterhalb der sogenannten minimalen Hemmkonzentration des Antibiotikums sind normalerweise keine oder nur sehr geringe cytoplasmatische Enzymaktivitäten unter den gewählten Testbedingungen nachzuweisen. Bei Antibiotikumkonzentrationen gleich oder über der minimalen Hemmkonzentration sind cytoplasmatische Enzymaktivitäten nachweisbar, die z. B. den durch eine Ultraschallbehandlung der Zellen freisetzbaren cytoplasmatischen Enzymaktivitäten entsprechen.

Bei den cytoplasmatischen Enzymen, die zur Empfindlichkeitsprüfung von Bakterien gegen β-Lactamantibiotika herangezogen werden, kann es sich um ubiquitär in Bakterien vorkommende Enzyme handeln oder um Enzyme, die spezifisch für einzelne Bakterienfamilien, Bakteriengattungen oder sogar Bakterienarten sind.

Bei gramnegativen Bakterien von medizinischem Interesse, die auf MacConkey-Agar als Selektivnährboden isoliert wurden, deutet beispielsweise eine positive β-Galactosidaseaktivität stets auf das Vorliegen eines Vertreters der Gattungen Escherichia, Citrobacter, Klebsiella, Enterobacter, Hafnia, Serratia oder Arizona hin.

Bei der Untersuchung einer cytoplasmatischen Enzymaktivität, die für eine Bakterienart spezifisch ist, kann eine Empfindlichkeitsprüfung der entsprechenden Bakterienart in einer Mischkultur mit anderen Bakterienarten durchgeführt werden. Es entsteht somit keine Verzögerung der Durchführung einer Empfindlichkeitsprüfung des vermutlichen Krankheitserregers durch das Isolieren von Reinkulturen.

Escherichia coli zeigt z. B. im Gegensatz zu anderen coliformen Keimen meist eine starke cytoplasmatische β-D-Glucuronidase-Aktivität. Die Empfindlichkeit von Escherichia coli gegen β-Lactamantibiotika läßt sich somit in einer Mischkultur verschiedener coliformer Bakterien einfach und schnell bestimmen.

Das Testsystem hat folgende Zusammensetzung: ein gepuffertes Nährmedium mit verschiedenen Antibiotikumkonzentrationen, ein oder mehrere Enzymsubstrate, vorzugsweise fluorogene bzw. chromogene Substrate und gegebenenfalls ein oder mehrere Coenzyme (Cofaktoren) sowie gegebenenfalls mindestens einen Redoxindikator. Vorzugsweise enthält das Testsystem noch ein Detergenz. Zur Empfindlichkeitsprüfung von Bakterien gegen Antibiotika wird das Testsystem mit einer Suspension des zu untersuchenden Bakteriums beimpft.

Als gepufferte Nährmedien können z. B. Müller-Hinton-Bouillon, Hirn-Herz-Bouillon, Caseinpepton-Sojamehlpepton-Bouillon in geeigneten Puffern wie Phosphatpuffer, Iris-(hydroxymethyl)-aminomethan (Tris-Puffer), N-(2-Hydroxyethyl)-piperazin-N-2-ethansulfonsäure (HEPES-Puffer) oder anderen gebräuchlichen Puffersystemen eingesetzt werden. Ein bevorzugter Puffer ist ein 0,1 M Phosphatpuffer vom pH-Wert 7. Die Wahl des Puffers sowie des pH-Wertes richtet sich sowohl nach den optimalen Testbedingungen des nachzuweisenden Enzyms als auch nach den geeigneten physiologischen Bedingungen für die Bakterien. Der Fachmann kann die geeigneten Bedingungen nach Routinemethoden ermitteln.

Antibiotika mit Primärwirkort in der Mureinbiosynthese sind z. B. Penicilline, wie Penicillin G, Penicillin V, Ampicillin, Cephalosporine, wie Cephalosporin C, Cephaloridin, Cephalotin, Vancomycin, Cycloserin, Bacitracin. Die Endkonzentrationen der Antibiotika im Inkubationsansatz sollten steigend in einem Bereich von 0 - 64 μg/ml liegen, vorzugsweise in einem Bereich bis zu 8 μg/ml.

Bei den in Gegenwart des Antibiotikums aus den Bakterien freigesetzten cytoplasmatischen Enzymaktivitäten, die am einfachsten nachgewiesen werden können, handelt es sich vorzugsweise um hydrolytische Enzyme (Hydrolasen), z. B. Glycosidasen wie β-D-Galaktosidase und β-D-Glucuronidase oder Oxidoreduktasen wie Succinatdehydrogenase und Malatdehydrogenase.

Die Enzymaktivitäten von Glycosidasen werden beispielsweise mit Hilfe fluorogener oder chromogener Substrate bestimmt. Solche Substrate sind z. B. für die β-D-Galactosidase: α- bzw. β-Naphthyl-β-D-galactopyranosid, o- bzw. p-Nitrophenyl-β-D-galactopyranosid, 4-Methylumbelliferyl-β-D-galactopyranosid; für die β-D-Glucuronidase: α- bzw. β-Naphthyl-β-D-glucuronid, o- bzw. p-Nitrophenyl-β-D-glucuronid, 4-Methylumbelliferyl-β-D-glucuronid. Die cytoplasmatischen Enzymaktivitäten der Oxidoreduktasen werden in üblicher Weise anhand der entsprechenden Enzymsubstrate und Coenzyme bestimmt, wobei auch fluorogene oder chromogene Redoxreaktionen nachgeschaltet werden können. Geeignete Coenzyme sind z. B. NAD, NADP, bzw. die reduzierten Formen NADH und NADPH. Geeignete Redoxindikatoren sind solche, deren oxidierte und reduzierte Form visuell, photometrisch oder mit elektrochemischen Verfahren eindeutig zu unterscheiden sind, z. B. Tetrazoliumsalze wie 3-(4-Jodphenyl)-2-(4-nitrophenyl-5-phenyl-2H-tetrazoliumchlorid und 3-(4,5-Di-methylthiazol-2-yl)-2,5-diphenyl-2H-tetrazoliumbromid. Je nach Art der Redoxindikatoren kann die Übertragung der Elektronen direkt oder mit Hilfe eines sogenannten Elektronenüberträgers erfolgen. Als soche kommen z. B. Phenazinmethosulfat, Phenazinethosulfat, Meldolablau, Diaphorase in Frage.

Die für das jeweilige Enzym geeigneten Substrate und Testbedingungen sind aus der Literatur bekannt oder können nach Standardverfahren ermittelt werden.

Die Enzymsubstrate werden üblicherweise in Konzentrationen von $10^{-1}$ bis $10^{-5}$ M eingesetzt.

Die auf Antibiotikaempfindlichkeit zu prüfenden Bakterien werden z. B. als Reinkulturen eingesetzt, die von Blutagar, Caseinpepton-Sojamehlpepton-Agar, Brolacin-Agar oder ähnlichen Nährböden gewonnen wurden. Die Bakteriensuspension im Testansatz sollte einen Keimgehalt von $10^5$ - $10^8$ Keime/ml besitzen.

Vorzugsweise enthält das Mittel nach der Erfindung kationenaktive, anionenaktive und/oder nichtionogene Detergenzien. Diese sind hinsichtlich ihrer Art nicht kritisch und können aus den üblicherweise verwendeten Detergenzien ausgewählt werden. Bewährt haben sich z. B. Polyoxyethylenderivate der Sorbitanester wie Polyoxyethylensorbitanmonolaurat, -sorbitanmonopalmitat und -sorbitanmonooleat. Diese werden in Konzentrationen von $10^{-4}$ bis $10^{-6}$ M eingesetzt; bei diesen Detergenzkonzentrationen werden aus Zellen mit intaktem Mureingerüst keine signifikanten Mengen an cytoplasmatischen Enzymen freigesetzt.

Das Testsystem besteht vorzugsweise aus einem Tiefziehteil mit zahlreichen, etwa 100 μl großen Kavitäten, aus einer handelsüblichen Mikrotiterplatte bzw. aus einem speziellen Küvettenriegel; die Reagenzien des Testsystems liegen zweckmäßigerweise in lyophilisierter Form vor.

Die Erfindung wird durch die folgenden Beispiele näher erläutert:

**Beispiel 1**

Eine etwa 1 mm große Kolonie einer 12 - 18 Stunden alten Kultur (Übernachtkultur) von Enterobacter cloacae auf Brolacin-Agar wird in 1,0 ml 0,1 M Phosphatpuffer, pH 7,0, suspendiert. Jeweils 100 μl der Bakteriensuspension werden zu 100 μl Testlösung mit verschiedenen Konzentrationen an Ampicillin (D(-)-α-Aminobenzylpenicillin) in die Kavitäten einer Mikrotiterplatte gegeben. Die Endkonzentrationen an Ampicillin im Inkubationsansatz sind 0 - 0,5 - 1,0 - 2,0 - 4,0 - 8,0 μg/ml.

Die Testlösung besteht aus doppelt konzentrierter Müller-Hinton-Bouillon in 0,1 M Phosphatpuffer, pH 7,0 die 2 x $10^{-5}$ M an 4-Methylumbelliferyl-β-D-galactopyranosid sowie 2 x $10^{-5}$ M an Octylphenol-polyethylenglykolether ist und die oben genannten Ampicillinkonzentrationen enthält. Nach 30 Minuten Inkubation des Testansatzes bei 37° C wird die Mikrotiterplatte mit einer UV-Lampe bei 366 nm bestrahlt. Eine Hemmung der Mureinbiosynthese durch Ampicillin ist an einer Fluoreszenz des Testansatzes infolge signifikanter β-D-Galaktosidaseaktivität im Vergleich zur Kontrolle ohne Antibiotikum bzw. Ansätzen mit Antibiotikumkonzentrationen unterhalb der minimalen Hemmkonzentration zu erkennen. Die Testansätze mit 4,0 und 8,0 μg/ml Ampicillin zeigen eine deutliche Fluoreszenz, d. h. bei diesen Ampicillinkonzentrationen wird die Mureinbiosynthese von Enterobacter cloacae unter den gewählten Testbedingungen gehemmt.

**Beispiel 2**

Von einer Übernachtkultur von Escherichia coli auf Blutagar wird eine Bakteriensuspension mit etwa $10^7$ Keimen/ml in 0,1 M Phosphatpuffer, pH 7,0, hergestellt. Jeweils 100 μl der Bakteriensuspension werden zu 100 μl Testlösung in die Kavitäten einer Mikrotiterplatte gegeben, die verschiedene Cycloserinkonzentrationen enthält. Die Endkonzentration an Cycloserin im Testansatz ist 0 - 0,5 - 1,0 - 2,0 - 4,0 - 8,0 μg/ml.

Die Testlösung ist eine doppelt konzentrierte Müller-Hinton-Bouillon in 0,1 M Phosphatpuffer, pH 7,0, die 2 x $10^{-5}$ M an 4-Methylumbelliferyl-β-D-glucuronid sowie 2 x $10^{-5}$ M an Octylphenol-polyethylenglokolether ist und die oben genannten Cycloserinkonzentrationen enthält. Nach 30 Minuten Inkubation des Testansatzes bei 37°C wird die Mikrotiterplatte mit einer UV-Lampe bei 366 nm bestrahlt. Die Testansätze mit 4,0 und 8,0 µg/ml Cycloserin zeigen eine deutliche Fluoreszenz infolge signifikanter β-D-Glucuronidaseaktivität, d. h. die minimale Hemmkonzentration von Cycloserin beträgt bei Escherichia coli unter den gewählten Testbedingungen 4,0 µg/ml.

### Beispiel 3

10 etwa 1 mm große Kolonien einer 16 - 20 Stunden alten Kultur von Citrobacter freundii auf Blutagar werden in 5 ml 0,1 M Phosphatpuffer, pH 7,4 suspendiert. Jeweils 0,5 ml der Bakteriensuspension werden zu 0,5 ml Testlösung mit verschiedenen Konzentrationen an Ampicillin gegeben. Die Endkonzentrationen an Ampicillin im Inkubationsansatz sind 0 - 0,5 - 1,0 - 2,0 - 4,0 - 8,0 - 16,0 µg/ml.

Die Testlösung besteht aus doppelt konzentrierter Müller-Hinton-Bouillon in 0,1 M Phosphatpuffer, pH 7,4, die $2.10^{-3}$ M an Oxalacetat, $4.10^{-4}$ M an NADH sowie $2.10^{-5}$ M an Octylphenol-polyethylenglykolether ist. Nach 45 Minuten Inkubation des Testansatzes bei 37°C wird die Malatdehydrogenaseaktivität durch Bestimmung des NADH-Gehalts (Extinktionsmessung bei 340 nm) gemessen.

Eine Hemmung der Mureinbiosynthese durch Ampicillin ist an einer deutlich geringeren Extinktion des Testansatzes bei 340 nm im Vergleich zur Kontrolle ohne Antibiotikum bzw. Ansätzen mit Antibiotikumkonzentrationen unterhalb der minimalen Hemmkonzentration zu erkennen. Die Testansätze mit 8,0 und 16,0 µg/ml Ampicillin zeigen eine deutlich geringere Extinktion bei 340 nm, d. h. in diesen Testansätzen kann eine signifikante Malatdehydrogenaseaktivität nachgewiesen werden. Unter den gewählten Testbedingungen wird die Mureinbiosynthese bei Citrobacter freundii bei 8,0 und 16,0 µ g/ml Ampicillin gehemmt.

### Patentansprüche

1. Verfahren zur Empfindlichkeitsprüfung von Bakterien gegen Antibiotika mit Primärwirkung in der Mureinbiosynthese, dadurch gekennzeichnet, daß die in Gegenwart von Antibiotikum und Enzymsubstrat aus den Bakterien freigesetzten cytoplasmatischen Enzymaktivitäten direkt bestimmt werden.

2. Verfahren nach Anspruch 1, dadurch gekennzeichnet, daß die cytoplasmatischen Aktivitäten hydrolytischer Enzyme anhand fluorogener oder chromogener Enzymsubstrate bestimmt werden.

3. Verfahren nach Anspruch 1, dadurch gekennzeichnet, daß die cytoplasmatischen Aktivitäten von Oxidoreduktasen anhand von Enzymsubstraten, Coenzymen und gegebenenfalls fluorogenen oder chromogenen Redoxindikatoren bestimmt werden.

### Claims

1. Process for testing the sensitivity of bacteria towards antibiotics with a primary action in murein biosynthesis, characterised in that the cytoplasmic enzyme activities released from the bacteria in the presence of the antibiotic and enzyme substrate are determined directly.

2. Process according to Claim 1, characterised in that the cytoplasmic activities of hydrolytic enzymes are determined with the aid of fluorogenic or chromogenic enzyme substrates.

3. Process according to Claim 1, characterised in that the cytoplasmic activities of oxidoreductases are determined with the aid of enzyme substrates, coenzymes and, if appropriate, fluorogenic or chromogenic redox indicators.

### Revendications

1. Procédé pour déterminer la sensibilité des bactéries aux antibiotiques ayant leur effet primaire sur la biosynthèse de la muréine, caractérisé en ce que les activités enzymatiques cytoplasmiques libérées par les bactéries en présence d'antibiotiques et de substrats enzymatiques sont déterminées directement.

2. Procédé selon la revendication 1, caractérisé en ce que les activités cytoplasmiques des enzymes hydrolytiques sont déterminées à l'aide de substrats enzymatiques fluorescents ou chromogènes.

3. Procédé selon la revendication 1, caractérisé en ce que les activités cytoplasmiques des oxydoréductases sont déterminées à l'aide de substrats enzymatiques, les coenzymes et, éventuellement, les indicateurs redox fluorescents ou chromogènes.